(19) Europäisches Patentamt
European Patent Office
Office européen des brevets

(11) Publication number: **0 005 882**
**B1**

(12) **EUROPEAN PATENT SPECIFICATION**

(45) Date of publication of patent specification: **08.06.83**

(21) Application number: **79200256.0**

(22) Date of filing: **23.05.79**

(51) Int. Cl.³: **C 07 C 131/02,** A 01 N 53/00, C 07 D 307/42

(54) **A method of combating acarine pests by means of oxyimino-substituted cyclopropane-carboxylate esters, Z-isomers of certain such esters.**

(30) Priority: **02.06.78 US 911743**

(43) Date of publication of application:
**12.12.79 Bulletin 79/25**

(45) Publication of the grant of the patent:
**08.06.83 Bulletin 83/23**

(84) Designated Contracting States:
**AT BE CH DE FR GB IT LU NL SE**

(56) References cited:
**FR - A - 2 211 454**
**FR - A - 2 240 914**

**Nature, Vol. 248, p. 710—711 (1974)**
**J. Chem. Soc. 1975, p. 1865**
**Pesticide Formulations, 1973, Chap. 1, p. 38—42**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Roman, Steven Alan**
**10854 Olive Avenue**
**Oakdale California 95361 (US)**
Inventor: **Soloway, Samuel Barney**
**3401 Mansfield Lane**
**Modesto California 95350 (US)**

(74) Representative: **Rogers, Roy Charles et al,**
**4 York Road**
**London SE1 7NA (GB)**

**EP 0 005 882 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

A method of combating acarine pests by means of oxyimino-substituted cyclopropane-carboxylate esters, Ż-isomers of certain such esters

This invention relates to oxyimino-substituted cyclopropanecarboxylate esters, to a process for their manufacture, to compositions containing the esters, to their use as pesticides, and to certain novel intermediates.

US Patent No. 3,922,269 discloses a class of 2,2-dimethyl-3-(oxyiminomethyl)cyclopropanecarboxylic acid esters useful as insecticides. The existence of a variety of optical and geometric isomers of such compounds is discussed in relation to configuration about the cyclopropane ring atoms and configuration of the alcohol moiety of the ester. In particular it is stated in the passage at column 4 line 65 to column 5 line 30 that esters in which the two hydrogen atoms on the cyclopropane ring are in the absolute stereochemical relationship equivalent to that in (+)-*trans*-chrysanthemic acid, i.e. (1R,trans)-chrysanthemic acid, tend to be among the most insecticidally active of the various isomers and are preferred for that reason, although the (1R,cis)- are also said to be very active. However only (1R,trans)-cyclopropanecarboxylate oximes form the basis of the working examples in US Patent No. 3,922,269.

It has now been found that certain oxyimino-substituted cyclopropanecarboxylate esters are useful pesticides (insecticides and acaricides) and exhibit high knockdown characteristics.

The invention provides (1R, *cis*)-oxyimino-substituted-cyclopropanecarboxylate esters, substantially free of other stereoisomers, and having the formula

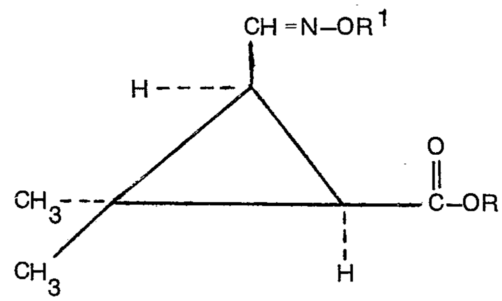

(A)

wherein $R^1$ represents a hydrogen atom, an alkyl group containing from 1 to 10 carbon atoms optionally-substituted by one or more halogen atoms, a (cycloalkyl)alkyl group containing 3 to 7 ring carbon atoms, a total of 4 to 9 carbon atoms and optionally ring-substituted by one or more halogen atoms, a cycloalkyl group containing 3 to 7 ring carbon atoms, an alkenyl group containing 2 to 4 carbon atoms optionally substituted by one or more halogen atoms or an alkynyl group containing 2 to 4 carbon atoms or an aryl group containing 6 to 12 carbon atoms or an aralkyl group containing 7 to 10 carbon atoms, each optionally ring- substituted by one or more halogen atoms; R is a group of the formula

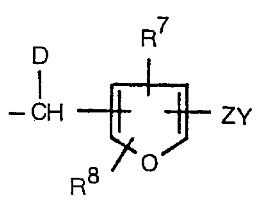

or

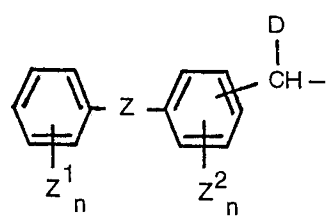

I

IV

wherein Y represents a hydrogen atom or a phenyl group which is unsubstituted or substituted in the ring by methyl, methoxy or chlorine, $R^7$ and $R^8$, which may be the same or different, each represents a hydrogen atom or a methyl group, D represents H, —CN, —C≡CH or

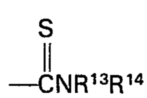

in which $R^{13}$ and $R^{14}$ may be the same or different, each represent a hydrogen atom or an alkyl group containing from 1 to 10 carbon atoms, Z represents $—CH_2—$, —O—, —CO—, or —S—, $Z^1$ and $Z^2$, which may be the same or different, each represent halogen or an alkyl group containing 1 to 4 carbon atoms and n is 0, 1 or 2. It will be noted that when D is —CN, —C≡CH or

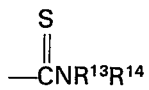

then the alcohol moiety of the ester can be in R,S-racemic or in the S- configuration.

In the above formulae, suitable halogen atom substituents are chlorine, fluorine or bromine.

Since the biological activity of various optical or geometric isomers and diastereoisomer pairs within the (1R,*cis*)-esters of the invention may differ somewhat, it may be desirable to use a more active optical and/or geometric isomer or diastereoisomer pair of the invention substantially free of the other isomers or pair.

The oxime substituent group of the esters according to the invention gives rise to geometric isomerism by virtue of the presence of an asymmetrically-substituted double bond. These isomers are usually described as follows:

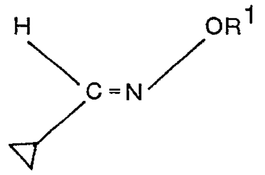

anti- or E-isomer

and

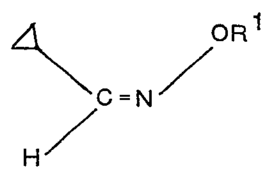

syn- or Z-isomer

A useful subclass of the invention comprises (1R,*cis*)-esters in which the oxime substituent is in the Z-isomer form as such isomers can be several times more pesticidally-active than when the oxime substituent is in the E-isomer form or is a mixture of the E- and Z- isomer forms.

It should be noted that esters wherein $R^1$ is haloalkyl, (cycloalkyl)alkyl optionally-substituted by halogen, haloalkenyl, alkynyl, aryl optionally-substituted by halogen, or aralkyl optionally-substituted by halogen are also novel in the (1R,*trans*) or racemic form as for example alpha-cyano-3-phenoxybenzyl (1R,*trans*)-2,2-dimethyl-3-((cyclopropylmethoxyimino)methyl)-cyclopropanecarboxylate, 3-phenoxybenzyl (1R,*trans*)-2,2-dimethyl-3-((cyclopropylmethoxyimino)methyl)cyclopropanecarboxylate and alpha-cyano-3-phenoxybenzyl (1R,*trans*)-2,2-dimethyl-3-((cyclo-pentoxyimino)methyl)cyclopropanecarboxylate.

When the (1R,*cis*)ester is one formally derivable from a furylmethyl alcohol, it is preferred that the furylmethyl alcohol be a 3-furylmethyl alcohol as described and claimed in U.S. Patent No. 3,466,304.

In the furylmethyl alcohols (A; R = I), and particularly in the 3-furyl-methyl alcohols, it is preferred that $R^7$ and $R^8$ each represent hydrogen or groups containing up to 4 carbon atoms, particularly a methyl group and that Y represents a phenyl group which is unsubstituted or substituted in the ring by a group containing up to 4 carbon atoms, e.g., methyl or methoxy, or by chlorine and Z is $—CH_2—$ and D is H. Analogues of these compounds where Z is —O—, —S— or —CO— and D is CN or C≡CH are also of interest. Further compounds of interest are those where Y represents a hydrogen atom, an alkyl group containing up to 4 carbon atoms, an alkenyl group containing up to 4 carbon atoms, e.g. vinyl, an alkadienyl group containing up to 4 carbon atoms or an alkynyl group, e.g. propargyl, or a furyl group.

Specific alcohols of this category, from which the (1R,*cis*)-esters of the invention are formally derivable, include 5-benzyl-3-furylmethyl alcohol, 5-benzyl-2-methyl-3-furylmethyl alcohol, 5-benzyl-3-furylmethyl alcohol, 4-benzyl-5-methyl-2-furylmethyl alcohol, 5-(p-methylbenzyl)methyl-3-furylmethyl alcohol, 2,4,5-trimethyl-3-furylmethyl alcohol and 4,5-dimethyl-2-furylmethyl alcohol, 5-phenoxy and 5-benzoyl-3-furylmethyl alcohol and alpha-cyano substituted 5-benzyl-5-benzoyl- or 5-phenoxy-3-furylmethyl alcohol.

The cyclopentenolones from which the (1R,*cis*)-esters of the invention are formally derivable are those unsubstituted in the 3-position or those substituted in the 3-position by a methyl group ($R^9$ = H or $CH_3$).

The cyclopentenolones (A; R = II) unsubstituted in the 2-position are described and claimed in U.S. Patent No. 3,720,703.

Some of these alcohols are the 3-desmethyl analogues of the alcohols from which the naturally occurring pyrethrins are derived. In the present invention, it is preferred that $R^{10}$ and $R^{11}$ each represent hydrogen, methyl or ethyl and $R^{12}$ represents an aryl group such as a phenyl group or a phenyl group substituted by a halogeno or alkyl or alkoxy substituent of 1 to 4 carbon atoms, for example tolyl, xylyl, p-chlorophenyl or p-methoxy-phenyl. $R^{12}$ may also represent a 2- or 3-furyl group or an alkenyl group such as vinyl, 1-propenyl or 1,3-butadienyl group.

When the (1R,*cis*)-esters of the invention are formally derivable from the cyclopentenolones which are substituted in the 3-position by the methyl group ($R^9$ = methyl), the (1R,*cis*)-esters may be derived from allethrolone ($R^{10} = R^{11}$ = H, $R^{12}$ = vinyl), pyrethrolone ($R^{10} = R^{11}$ = H, $R^{12}$ = 1,3-

butadienyl), cinerolone ($R^{10} = R^{11} = H$, $R^{12}$ = 1-propenyl), jasmolone ($R^{10} = R^{11} = H$, $R^{12}$ = 1-butenyl), or furethrolone ($R^{10} = R^{11} = H$, $R^{12}$ = 2-furyl).

When the (1R,*cis*)-esters of the invention are phthalimido-methyl esters where R is of formula III, they may be phthalimido, dihydrophthalimido or tetrahydrophthalimidomethyl esters where the phthalimido, dihydrophthalimido or tetrahydrophthalimido residue (R is formula III) is one described in British Pat. Specifications Nos. 985,006, 1,052,119 or 1,058,309. 3,4,5,6-Tetrahydrophthalimidomethyl esters are of particular interest.

When the (1R,*cis*)-esters of the invention are those where R is of formula IV, it is preferred that they be 3-benzylbenzyl esters, 3-benzoylbenzyl esters, or 3-phenoxybenzyl esters although each of the rings may be substituted by up to 3 chloro and/or methyl groups. Other esters of particular interest where R is of formula IV are those where Z represents —O— or —$CH_2$— and D represents CN or C≡CH, e.g. esters of alpha-cyano or alpha-ethynyl substituted 3-phenoxy-, 3-benzyl or 3-benzyloxybenzyl alcohol. Such alcohols are described in U.S. Patents 3,666,789, 3,835,176 and 3,862,174.

Variations in activity, of course, depend on the individual combinations of R and $R^1$ and are dependent upon the susceptibility of an individual pest to certain structural subtleties in the various combinations of R and $R^1$.

Typical examples of compounds within the scope of the invention are:

alpha-ethynyl-5-benzyl-3-furylmethyl (1R,*cis*)-2,2-dimethyl-3-((sec-butoxyimino)methyl)-cyclopropanecarboxylate alpha-cyano-3-phenoxybenzyl (1R,*cis*)-2,2-dimethyl-3-((propargyl-oxyimino)-methyl)-cyclopropanecarboxylate 3-phenoxybenzyl (1R,*cis*)-2,2-dimethyl-3-((isobutoxyimino)-methyl-cyclopropanecarboxylate
alpha-thiocarbamoyl-3-benzylbenzyl (1R,*cis*)-2,2-dimethyl-3-(trifluoromethoxyimino)methyl)-cyclopropanecarboxylate 3-phenoxybenzyl (1R,*cis*)-2,2-dimethyl-3-(p-chlorophenoxy-imino)-methyl)cyclopropanecarboxylate.

Because of their pesticidal utility in agricultural and domestic locations, preferred esters according to the invention are those of formula A in which R is a group of formula I or IV (i.e. those wherein R is 5-benzyl-3-furylmethyl, alpha-cyano-3-phenoxybenzyl, 3-phenoxybenzyl or alpha-ethynyl-3-phenoxy-benzyl) and $R^1$ is an alkyl group containing 1 to 6 carbon atoms, a (cycloalkyl)alkyl group containing 3 to 6 ring carbon atoms, a total of 4 to 8 carbon atoms and optionally ring-substituted by 1 to 4 chlorine, fluorine and/or bromine atoms, a cycloalkyl group containing 3 to 6 ring carbon atoms, an alkenyl group containing 2 to 4 carbon atoms, optionally substituted by one or two chlorine, fluorine and/or bromine atoms or an alkynyl group containing 2 to 4 carbon atoms, an aryl group containing 6 to 12 carbon atoms or an aralkyl group containing 7 to 10 carbon atoms.

In the pestidical (1R,*cis*)-esters of the invention, $R^1$ preferably represents an alkyl group containing 2 to 6 carbon atoms, such as ethyl, n-propyl, isopropyl, n-butyl, sec-butyl, isobutyl, tert-butyl, amyl, isoamyl, tert-amyl and n-hexyl, a (cycloalkyl)alkyl group containing 3 to 6 ring carbon atoms and a total of 4 to 8 carbon atoms, such as cyclopropylmethyl, 1-(cyclopropyl)-ethyl, and cyclohexylmethyl, a cycloalkyl group containing 3 to 6 ring carbon atoms, such as cyclopropyl, cyclobutyl, cyclopentyl and cyclohexyl, an alkenyl or alkynyl group containing 2 to 4 carbon atoms such as allyl or propargyl, an aryl group containing from 6 to 10 carbon atoms such as phenyl or an aralkyl group containing from 7 to 10 carbon atoms, such as benzyl, or phenethyl.

Preferred because of their pesticidal properties are those (1R,*cis*)-esters wherein $R^1$ is an alkyl group containing 2 to 6 carbon atoms, a (cycloalkyl)alkyl or cycloalkyl group containing 4 or 5 carbon atoms, allyl, phenyl or benzyl. Particularly suitable are those compounds wherein $R^1$ is an alkyl, (cyclopropyl)alkyl or cycloalkyl group containing 4 or 5 carbon atoms, particularly n-butyl, isobutyl, tert-butyl, n-pentyl, cyclopentyl, or (cyclo-propyl)methyl.

Because of their pesticidal activity and ease of preparation, one preferred subclass of the invention are those esters wherein R is derived from 3-phenoxybenzyl alcohol. Examples of some highly active compounds of this subclass of the invention are:

3 - phenoxybenzyl (1R,*cis*) - 2,2 - dimethyl - 3 - ((cyclopropylmethoxyimino)methyl)cyclopropanecarboxylate and 3 - phenoxybenzyl (1R,*cis*) - 2,2 - dimethyl - 3 - ((isobutoxyimino)-methyl)cyclopropanecarboxylate.

Because of their pesticidal activity, another preferred subclass of the invention are those (1R,*cis*)-esters derived from alpha-cyano-3-phenoxybenzyl alcohol, particularly in the S- configuration. In fact, the (1R,*cis*)-esters wherein the alcohol substituent is R-alpha-cyano-3-phenoxybenzyl alcohol are without practical insecticidal utility. Thus, one preferred embodiment of the invention is directed to those (1R,*cis*)-esters derived from alpha-cyano-3-phenoxybenzyl alcohol in which such alcohol is in a racemic or, alternatively, in an S- configuration. Examples of some highly-active esters of this subclass of the invention are the following compounds wherein R is derived from alpha-cyano-3-phenoxybenzyl alcohol and $R^1$ is an alkyl, (cycloalkyl)alkyl or cycloalkyl group containing 4 or 5 carbon atoms:

alpha - cyano - 3 - phenoxybenzyl (1R,*cis*) - 2,2 - dimethyl - 3 - ((cyclopropylmethoxyimino)-methyl)cyclopropanecarboxylate,
alpha - cyano - 3 - phenoxybenzyl (1R,*cis*) - 2,2 - dimethyl - 3 - ((n - butoxyimino)methyl)cyclopropanecarboxylate,
alpha - cyano - 3 - phenoxybenzyl (1R,*cis*) - 2,2 - dimethyl - 3 - ((isobutoxyimino)methyl)cyclopropanecarboxylate.
alpha - cyano - 3 - phenoxybenzyl (1R,*cis*) - 2,2 - dimethyl - 3 - ((tert - butoxyimino)methyl)cyclopropanecarboxylate,
alpha - cyano - 3 - phenoxybenzyl (1R,*cis*) - 2,2 - dimethyl - 3 - ((n - pentoxyimino)methyl)cyclopropanecarboxylate,
and
alpha - cyano - 3 - phenoxybenzyl (1R,*cis*) - 2,2 - dimethyl - 3 - ((cyclopentoxyimino)methyl)-cyclopropanecarboxylate

which compounds are pesticidally-active when the alcohol moiety is present in the R,S-racemic or S-form, and the oxime substituent is in the Z- or S- isomer form or in a mixture of the E- and Z- isomer forms.

A particularly preferred subclass of the invention are those (1R,*cis*)-esters derived from the S-alpha-cyano-3-phenoxybenzyl alcohol and wherein the oxime substituent is the Z-isomer form.

It has been generally observed that esters, wherein the oxime substituent is in the Z-isomer form, are the most pesticidally active. Therefore, the Z-isomers (or mixtures of isomers in which the Z-isomer predominates) form another preferred subclass of the (1R,*cis*)-esters of the invention. That the Z-isomer form is more pesticidally active contrasts with another class of oxime pesticides disclosed in U.S. Patent 4,079,149, in which the E-isomer form is said to be the more pesticidally active of the oxime geometric isomer forms.

Because of their useful pesticidal properties, one preferred class of (1R,*cis*)-esters of the invention comprise those compounds wherein $R^1$ is (cycloalkyl)alkyl and R is one of the groups of formulae I—VI. Particularly preferred are (1R,*cis*)-esters wherein $R^1$ is cyclopropylmethyl, and R is 3-phenoxybenzyl or alpha-cyano-3-phenoxybenzyl or individual diastereoisomers of such (1R,*cis*)-esters.

According to a feature of the present invention the (1R,*cis*)-esters of general formula A may be prepared by esterification involving the reaction of an alcohol or derivative thereof of formula RQ, e.g. a compound formula

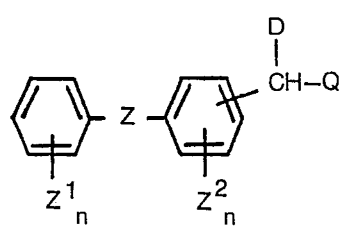

VII

with a (1R,*cis*)-cyclopropane carboxylic acid or derivative thereof of formula

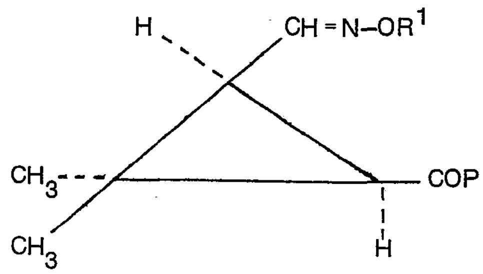

VIII

where Q and P are functional groups or atoms which will react to form an ester linkage and R, $R^1$, $Z^1$, $Z^2$, n and D are as defined above. Preferably Q and P are selected from halide, —OH and —OM where M is a cation.

It is usually convenient in practice either to treat the acid or acid halide with the alcohol (P = OH or halide and Q = OH) or to treat a halogeno compound (Q = halide) with a salt of the carboxylic acid (P = O—M where M is, for example a silver or triethylammonium cation).

Transesterification is not always practical and, it is useful to prepare the intermediate (1R,*cis*)-alkyl ester as a tert-butyl ester (R = tert-butyl) which can be selectively converted (under acid conditions) to give the free acid which can, after conversion to the acid halide, be esterified to a pesticidal (1R,*cis*)-ester.

Suitable routes to the (1R,*cis*)-esters in which D is

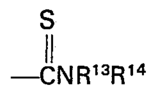

are similar to those described in Belgian patent 839,360. One route involves treating the corresponding nitrile (D is —CN) with hydrogen sulfide in the presence of a basic catalyst, preferably in the presence of a solvent. Useful solvents are lower alkanols, pyridine, or preferably a dipolar aprotic solvent, such as dimethylformamide or hexamethylphosphoramide. The catalyst is preferably a strong nitrogenous base, particularly a tertiary amine such as triethylamine, trimethylamine, or the like, or an alkanolamine, such as triethanolamine and the like. The reaction can be carried out at room temperature.

It is desirable that the reaction solution be saturated with hydrogen sulfide.

The (1R,*cis*)-alkyl esters of the present invention can also be prepared by treating an ester of (1R,*cis*)-caronaldehyde of formula IX

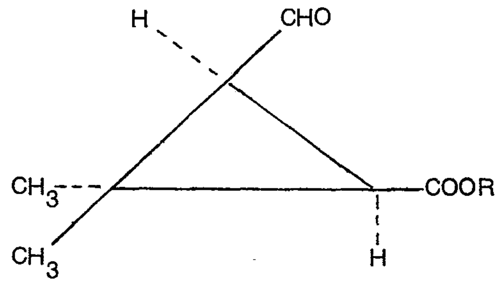

IX

where R is an alkyl group, with hydroxylamine or an O-substituted hydroxylamine of formula $R^1ONH_2$ where $R^1$ is as defined above, and in the case where $R^1$ represents hydrogen, subsequently hydrocarbylating the resulting oxime, if desired, with an alkyl (or alkenyl) halide or the like, to give an alkoxime (or alkenyloxime), etc. Oxime formation can take place by treating substantially equimolar amounts of aldehyde and hydroxylamine or hydrocarbyloxyamine in a polar solvent such as an alkanol, e.g. ethanol or dioxane. When the aldehyde is converted into the oxime by reaction with hydroxylamine and it is desired to convert the resulting oxime into an alkylated or alkenylated derivative or the like, this reaction may be carried out by procedures customarily used for the alkylation of phenols. Thus, the oxime may be treated in a polar solvent, such as ethanol, with an alkyl halide, typically the bromide, in the presence of a hydrogen halide acceptor and the mixture heated until reaction is complete.

Oxime formation is normally carried out using an acid addition salt of hydroxylamine or the hydrocarbyloxyamine, e.g. the hydrochloride. In the cases where it is desired to prepare a (1R,*cis*)-compound where $R^1$ represents methyl, the availability of methoxylamine hydrochloride makes it generally more convenient to carry out the reaction in one step using methoxylamine hydrochloride, but when compounds are required where $R^1$ represents a larger group, it is usually more convenient to form the oxime first and subsequently to hydrocarbylate the oxime.

Alcohols and halides of formula RQ (where R = a group of formula II) are described and claimed in U.S. Patent No. 3,720,703.

Alcohols of formula RQ where R is a group of formula IV may be prepared by reduction of the corresponding acids or esters or aldehyde e.g. with hydride, or by conversion of the corresponding halide to an ester, e.g. by reaction with sodium acetate, followed by hydrolysis of the ester, or by reaction of formaldehyde with a Grignard reagent derived from the corresponding halide. The halides of formula RQ where R is a group of formula IV can be prepared by halomethylation of the compound

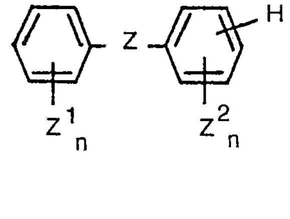

or side chain halogenation of

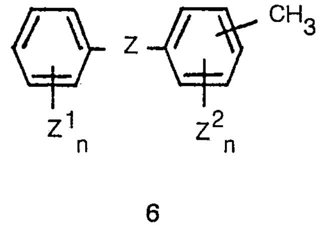

Alternatively, in another modification, the compounds of the invention are prepared by treating (1R,*cis*)-caronaldehydic acid previously described in U.S. Patent 3,723,469 and having the formula

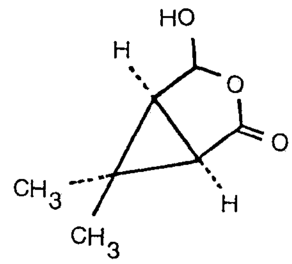

with an O-substituted hydroxylamine salt of the formula $R^1ONH_3$—W wherein $R^1$ is as defined above and W is the anion of salt-forming inorganic acid. Suitable inorganic acids include hydrohalogenic acids such as hydrochloric and hydrobromic, sulphur acids such as sulphuric, fluorosulfonic, phosphorus acids such as phosphoric, and nitrogen acids such as nitric acid, or boron acids such as boric or fluoboric acid.

The reaction is preferably conducted in an aqueous medium in the presence of a buffer, such as an alkali metal salt of a polybasic acid, including sodium hydrogen carbonate, potassium hydrogen tartrate, disodium hydrogen phosphate and the like. Generally, at least one mole of buffer is used for each mole of (1R,*cis*)-caronaldehydic acid.

The molar ratio of reactants is not critical and can be widely varied, generally a molar ratio of the O-substituted hydroxylamine salt to (1R,*cis*)-caronaldehydic acid is suitably from about 1.0 to about 1.5 and preferably from about 1.02 to about 1.3.

The reaction is generally conducted in the liquid phase by agitating, e.g. stirring, a mixture of the reactants. The resulting product is recovered by conventional techniques such as filtering, extracting or the like.

The reaction temperature is not critical and can easily range from ambient to the reflux temperature of any solvent employed at normal pressure. Generally, the temperature is between about 0°C to about 50°C.

A minor amount of co-solvent can be used in the reaction medium. Suitable co-solvents are lower alcohols containing from 1 to 6 carbon atoms, such as methanol, ethanol and the like.

The resulting (1R,*cis*)-acids are converted to the ester compounds of the invention, for example, by reaction with the arylmethyl halide, in the presence of triethylamine, in a solvent such as refluxing ethyl acetate.

An isomer mixture of the (1R,*cis*)-esters of the invention can be readily separated into the individual diastereoisomers using known procedures, as for example, by preparative scale liquid chromatography. One such chromatographic system which can be employed has the following characteristics:

Column — porisil polar bonded phase, 9.2 x 250 mm
Mobile Phase — 8% v/v diethyl ether in n-hexane
Flow Rate — 2.5 ml/min
Detection — $UV_{254}$ at 2.0 AUFS
Injection — typically 500 ml of a 20 mg/ml solution in the mobile phase.

Such a procedure readily yields the single diastereoisomers in greater than 90% purity (as determined by NMR analysis). In the case of (1R,*cis*)-esters of alpha-substituted alcohols four diastereoisomers are obtained.

Since it has been discovered that the (1R,*cis*)-esters of the invention in which the oxime substituent is in the Z-isomer form are pesticidally more active than when the oxime substituent is in either the E-isomer form or is a mixture of E- and Z-isomer forms, it can be desirable to convert the esters in E-isomer form into a mixture of esters in both the E- and Z-isomer forms. Such conversion is accomplished by the addition of a minor amount of an organic or inorganic acidic material. Any inorganic or organic acid or acidic acting material can be used, including acidic clays such as acidic silicates and aluminates or synthetic acidified clays, mineral acids such as hydrochloric or sulfuric acid, sulfonic acids such as toluenesulfonic acid, or organic acids, including lower alkanoic acids such as acetic, propionic or butyric acids. The acid can be used in a solid or liquid form. While the precise amount of acid used to convert the E-isomer or Z-isomer into the E- and Z-isomer mixture can vary depending on the particular oxyimino-substituted (1R,*cis*)-ester, from 0.001 to 5% by weight of acid based on the E-isomer or Z-isomer is generally sufficient. Preferably, from 0.01 to 5% by weight of acid is used.

The invention includes, within its scope, pesticidal compositions comprising a pesticidally acceptable adjuvant — that is, at least one carrier or a surface-active agent — and, as active ingredient,

at least one pesticidally active (1R,*cis*)-ester of this invention. Likewise, the invention includes also a method of combating insect, acarine or other arthropod pests at a locus which comprises applying to the pests or to the locus a pesticidally-effective amount of at least one compound of the invention.

With respect to the spectrum of pesticidal activity, the compounds of this invention exhibit a selective or non-selective activity on such orders as Coleoptera, Lepidoptera (especially larvae) Diptera, Orthoptera, Hemiptera, Homoptera and Acarina depending upon a specific combination of acid and an alcohol according to the present invention. The compositions according to the present invention are very useful for controlling disease carrying insects such as mosquitoes, flies and cockroaches, grain insects such as rice weevil (*Sitophilus oryzae*) and mites as well as agricultural noxious insects such as planthoppers, green rice leafhopper (*Nephotettix bipuntatus cinticeps* Uhler), diamond-back moths (*Plutella maculipennis* Curtis), imported cabbage worm (*Pieris rapae* Linne), rice stem borers (*Chilo suppressalis* Walker), corn earworm larvae (*Heliothis zea* Boddie), aphids, tortrixes, leaf-miners and the like.

The (1R,*cis*)-esters can be used for harvested crops, horticultural application, forests, cultures in green house, and packaging materials for foodstuffs.

The term "carrier" as used herein means a material, that may be inorganic or organic and of synthetic or natural origin with which the active compound is mixed or formulated to facilitate its application to the plant, seed, soil and other object to be treated, or its storage, transport or handling. The carrier may be a solid or a liquid.

Suitable solid carriers may be natural and synthetic clays and silicates, for example, natural silicas such as diatomaceous earths; magnesium silicates, for example, talcs; magnesium aluminium silicates, for example attapulgites and vermiculites; aluminium silicates, for example kaolinites, montmorillonites and micas; calcium carbonate; calcium sulfate; synthetic hydrated silicon oxides and synthetic calcium or aluminium silicates; elements such as for example, carbon and sulphur; natural and synthetic resins such as, for example, coumarone resins, polyvinyl chloride and styrene polymers and copolymers; solid polychlorophenols; bitumen, waxes such as beeswax, paraffin wax, and chlorinated mineral waxes; degradable organic solids, such as ground corn cobs and walnut shells; and solid fertilisers, for example superphosphates.

Suitable liquid carriers include solvents for the compounds of this invention and liquids in which the toxicant is insoluble or only slightly soluble.

Examples of such solvents and liquid carriers, generally, are water, alcohols, for example, isopropyl alcohol, ketones, such as acetone, methyl ethyl ketone, methyl isobutyl ketone and cyclohexanone; ethers; aromatic hydrocarbons such as benzene, toluene and xylene; petroleum fractions, such as kerosene, light mineral oils, chlorinated hydrocarbons, such as methylene chloride, perchlorethylene, trichloroethane, including liquified normally vaporous gaseous compounds. Mixtures of different liquids are often suitable.

If used, the surface-active agent may be an emulsifying agent or a dispersing agent or a wetting agent. It may be non-ionic, ionic or preferably, mixtures of both. Surface-active agents usually applied in formulating pesticides may be used. Examples of such surface-active agents are the sodium or calcium salts of polyacrylic acids and lignin sulfonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol: fatty acids salts of low molecular weight, mono-, di- and trialkyl-amines; condensates of these with ethylene oxide and/or propylene oxide; condensation products of fatty alcohols or alkyl phenols, for example, p-octylphenol or p-octylcresol, with ethylene oxide and/or propylene oxide; sulfates or sulfonates of these condensation products; alkali or alkaline earth metal salts, preferably sodium salts of sulfonated castor oil, and sodium alkylaryl sulfonates such as sodium dodecylbenzene sulfonate; and polymers of ethylene oxide and copolymers of ethylene oxide and propylene oxide.

The compositions of the invention may be formulated as wettable powders, dusts, granules, solutions, emulsifiable concentrates, emulsions, suspension concentrates or aerosols. Encapsulated formulations and controlled release formulations are also contemplated, as are bait formulations. Wettable powders are usually compounded to contain 25, 50 or 75% w of toxicant and usually contain, in addition to solid carrier, 3—10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing 1/2—10% w of toxicant. Granules may be manufactured by extrusion of plastics, agglomeration or impregnation techniques. Generally, granules will contain 1/2—25% w toxicant and 0—10% w of additives such as stabilisers, slow release modifiers and binding agents. Emulsifiable concentrates usually contain, in addition to the solvent, and when necessary, cosolvent, 10—50% w/v toxicant, 2—20% w/v emulsifiers and 0—20% w/v of appropriate additives such as stabilisers, penetrants and corrosion inhibitors. Suspension concentrates are compounded so as to obtain a stable, non-sedimenting, flowable product and usually contain 10—75% w toxicant, 0—5% w of dispersing agents, 0.1—10% w of suspending agents such as protective colloids and thixotropic agents, 0—10% w of appropriate additives such as defoamers, corrosion inhibitors, stabilisers, penetrants and stickers, and as a carrier, water or an organic liquid in which the toxicant is substantially

insoluble; certain organic additives or inorganic salts may be dissolved in the carrier to assist in preventing sedimentation or as anti-freeze agents for water.

Aqueous dispersions and emulsions, for example, compositions obtained by diluting a wettable powder or an emulsifiable concentrate according to the invention with water, also lie within the scope of the present invention.

The compositions of the invention can also contain other ingredients, for example, other compounds possessing pesticidal, herbicidal or fungicidal properties, or attractants, such as pheromones, attractive food ingredients, and the like, for use in baits and trap formulations.

Particularly useful compositions can be obtained by using a mixture of two or more kinds of the present compounds, or by the use of synergists, such as those known for use with the general class of "pyrethroid" compounds, especially alpha-2-(2-butoxyethoxy)ethoxy-4,5-methylene-dioxy-2-propyltoluene also known as piperonyl butoxide, 1,2-methylene-dioxy-4-2-(octylsulfinyl)-propyl benzene, 4-(3,4-methylenedioxyphenyl)-5-methyl-1,3-dioxane also known as safroxane, N-(2-ethyhexyl)bicyclo 2,2,1 hept-5-ene-2,3-dicarboximide, octachlorodipropyl ether, isobornyl thiocyanoacetate, and other synergists used for allethrin and pyrethrin. Useful compositions can be prepared with other biological chemicals including other cyclopropanecarboxylates, organic phosphate type insecticides and carbamate type insecticides.

The compositions of the invention are applied in sufficient amount to supply the effective dosage of toxicant at the locus to be protected. This dosage is dependent upon many factors, including the carrier employed, the method and conditions of application, whether the formulation is present at the locus in the form of an aerosol, or as a film, or as discrete particles, the thickness of film or size of particles, the insect or acarine species to be controlled and the like, proper consideration and resolution of these factors to provide the necessary dosage of active material at the locus being within the skill of those versed in the art. In general, however the effective dosage of toxicants of this invention at the locus to be protected — i.e. the applied dosage — is of the order of 0.01% to 0.5% based on the total weight of the formulation, though under some circumstances the effective concentration will be as little as 0.001% or as much as 2%, on the same basis.

The superior activity of the (1R,*cis*)-esters of the invention is usefully employed when such an ester is present in an amount substantially greater than that usually present in the racemate of an oxyimino substituted ester. Therefore, use of the (1R,*cis*)-esters of the invention in a form substantially free of other stereoisomers is preferred, for example in a (1R,*cis*)-isomer purity of greater than about 85%, preferably in a (1R,*cis*)-isomer purity greater than about 90% or even greater than 95%.

The invention also extends to novel intermediates for use in the manufacture of the oxyimino-substituted cyclopropane-carboxylate esters according to the invention; the intermediates have the following general formula:

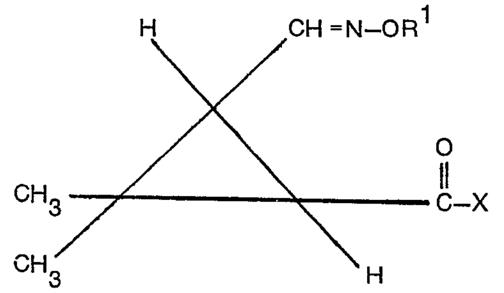

(B)

wherein $R^1$ has the meaning specified hereinbefore in relation to general formula A and X is chlorine, bromine or OR in which R represents a hydrogen atom, a salt-forming cation, or an alkyl group containing 1 to 20 carbon atoms. The intermediates are preferably in the (1R,*cis*)-configuration as they lead to the most active esters according to the invention.

When R is a salt-forming cation, it is preferably selected from alkali metals, alkaline earth metals, aluminium, heavy metals, such as copper, silver and nickel, ammonia or a tetrahydrocarbylammonium compound in which the total number of carbon atoms in the hydrocarbyl groups is between 4 and 70 carbon atoms. The hydrocarbyl groups can be alkyl, aryl or aralkyl. Preferably, the hydrocarbyl groups are selected from alkyl groups containing from 1 to 10 carbon atoms and aralkyl groups containing from 7 to 10 carbon atoms.

When R is an alkyl group, it preferably contains 1 to 20 carbon atoms, and especially 1 to 6 carbon atoms, for example, methyl, ethyl, n-propyl and tert-butyl.

These novel intermediates can be prepared by a process analogous to that employed for the manufacture of the (1R,*cis*)-esters of general formula A.

The invention is illustrated by the following examples which describe the preparation and biological testing of typical species of the invention with respect to representative insects and acarines. The identity of the products, including intermediates, was confirmed by elemental, infrared and nuclear magnetic resonance spectral (NMR) analyses as necessary.

Example 1
(1R,*cis*)-2,2-dimethyl-3-((isobutoxyimino)methyl)cyclopropanecarboxylic acid
(preparation of intermediate)

A solution of 1.7 g of (1R,*cis*)-caronaldehydic acid and 1.6 g of isobutoxyamine hydrochloride in 50 ml of water was stirred at room temperature for 5 hours in the presence of 2.2 g of sodium bicarbonate. The resulting mixture was filtered through celite, and the filtrate was acidified with concentrated hydrochloric acid, the resulting solution was extracted with methylene chloride, and the combined extracts were dried over magnesium sulfate and stripped to give 2.4 g of desired product as an oil; $[\alpha]_D^{25}$ + 33.2° ($CHCl_3$; c = 0.02 g/cc).

Example 2
Alpha-cyano-3-phenoxybenzyl (1R,*cis*)-2,2-dimethyl-3-(isobutoxyimino)methyl)-
cyclopropanecarboxylate

A solution of 2.1 g of (1R,*cis*)-2,2-dimethyl-3-(isobutoxyimino)methyl)cyclopropanecarboxylic acid as prepared in Example 1, 2.9 g of alpha-cyano-3-phenoxybenzyl bromide, 1 g of triethylamine and 25 ml of ethyl acetate was refluxed for about three hours and allowed to stand at room temperature for two days. The reaction mixture was diluted with methylene chloride and washed with water. The methylene chloride phase was then dried over magnesium sulfate and stripped to give 5 g of amber oil. This oil was chromatographed on a 3 foot silica gel column with a mixture of etherpentane (1:15 ratio) as the eluent to give a yellow oil. Rechromatographing this product with an 1:9 ratio of ether-pentane as the eluent gave 3.4 g of product as a yellow oil; $[\alpha]_D^{25}$ + 12.1° ($CHCl_3$; c = 0.02 g/cc).

Example 3
5-Benzyl-3-furylmethyl (1R,*cis*)-2,2-dimethyl-3-((methoxyimino)methyl)-
cyclopropanecarboxylate

A solution of 1.6 g of (5-benzyl-3-furyl)methyl chloride and 1.3 g of (1R,*cis*)-2,2-dimethyl-3-((methoxyimino)methyl)cyclopropanecarboxylic acid (prepared by a method similar to that shown in Example 1) and 0.8 g of triethylamine in 15 ml of ethyl acetate was refluxed for 30 hours. The resulting mixture was cooled, diluted with ether, and washed with water. The ether phase was dried over magnesium sulfate and stripped to give an oil. This oil was chromatographed on silica gel with a mixture of pentane-ether (6:1 ratio) as the eluant to give 1.5 g of desired product as a pale yellow oil; $[\alpha]_D^{25}$ + 5.4° ($CHCl_3$; c = 0.2 g/cc).

Example 4
3-phenoxybenzyl (1R,*cis*)-2,2-dimethyl-3-((allyloxyimino)methyl)cyclopropanecarboxylate

A solution of 3.7 g of 3-phenoxybenzyl bromide and 2.8 g of (1R,*cis*)-2,2-dimethyl-3-((allyloxyimino)methyl)cyclopropanecarboxylic acid (prepared by a method similar to that shown in Example 1) and 1.4 g of triethylamine in 30 ml of ethyl acetate was refluxed for 2 1/2 hours. The reaction mixture was diluted with ether and washed with water. The ether phase was dried over magnesium sulfate, filtered and stripped to give an oil. This oil in methylene chloride was passed through a florisil column to remove impurities to give 2.5 g of the desired product as an oil $[\alpha]_D^{25}$ +22.9° ($CHCl_3$; c = 0.012 g/cc).

Examples 5—20

Procedures similar to those of Examples 2—4 were used to prepare additional esters according to the invention shown in Table 1 below:

TABLE 1 – Oxyimino-substituted (1R, *cis*)-cyclopropanecarboxylates

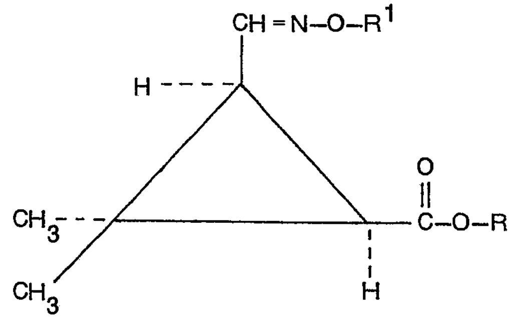

| Example | $R^1$ | R | E/Z Isomer | $[\alpha]^{25}_D$ ($CHCl_3$) |
|---|---|---|---|---|
| 5 | $-CH_3$ | alpha-cyano-3-phenoxybenzyl | E–Z–isomer | +17.9° |
| 6 | $-CH(CH_3)_2$ | alpha-cyano-3-phenoxybenzyl | E–Z–isomer | +19.6° |
| 7 | $-CH_2CH=CH_2$ | alpha-cyano-3-phenoxybenzyl | E–Z–isomer | +22.1° |
| 8 | –benzyl | alpha-cyano-3-phenoxybenzyl | E–Z–isomer | +30.0° |
| 9 | $-C_2H_5$ | alpha-cyano-3-phenoxybenzyl | E–Z–isomer | not det. |
| 10 | $-CH(CH_3)C_2H_5$ | alpha-cyano-3-phenoxybenzyl | E–Z–isomer | +23.8° |
| 11 | –phenyl | alpha-cyano-3-phenoxybenzyl | E–Z–isomer | not det. |
| 12 | $-CH_3$ | alpha-phenoxybenzyl | E–Z–isomer | +10.0° |
| 13 | $-n-C_3H_7$ | alpha-cyano-3-phenoxybenzyl | E–Z–isomer | +16.7° |
| 14 | $-n-C_4H_9$ | alpha-cyano-3-phenoxybenzyl | E–Z–isomer | +20.0° |
| 15 | $-(8CH_3)_3$ | alpha-cyano-3-phenoxybenzyl | E–Z–isomer | +17.5° |
| 16 | $-CH_2(CH_2)_4CH_3$ | alpha-cyano-3-phenoxybenzyl | E–Z–isomer | not det. |
| 17 | $-CH_2(CH_2)_4CH_3$ | alpha-cyano-3-phenoxybenzyl | E–Z–isomer | + 5.0° |
| 18a | $-CH_2CH(CH_3)_2$ | 3-phenoxybenzyl | E–Z–isomer | + 5.0° |
| 18b | $-CH_2CH(CH_3)_2$ | 3-phenoxybenzyl | E–isomer | +21.2° |

TABLE 1 — Oxyimino-sybstituted (1R, *cis*)-cyclopropanecarboxylates (Continued)

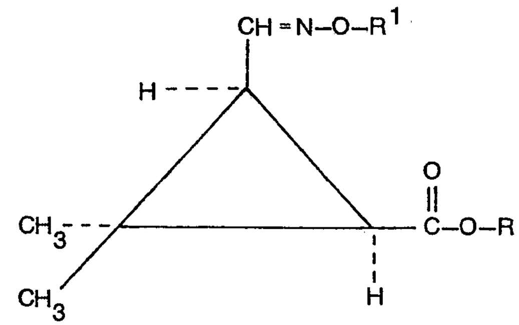

| Example | $R^1$ | R | E/Z Isomer | $[\alpha]_D^{25}$ $(CHCl_3)$ |
|---|---|---|---|---|
| 18c | $-CH_2CH(CH_3)_2$ | 3-phenoxybenzyl | Z–isomer | +23.8° |
| 19 | $-CH_2CH_2CH(CH_3)_2$ | alpha-cyano-3-phenoxybenzyl | E–Z–isomer | +10.0° |
| 20 | $-CH_2CH(CH_3)CH_2CH_3$ | alpha-cyano-3-phenoxybenzyl | E–Z–isomer | + 6.2° |

Example 21

(1R,*cis*)-2,2-dimethyl-3-((cyclopropylmethoxyimino)methyl)cyclopropanecarboxylic acid (preparation of intermediate)

A solution of 1.7 g of (1R,*cis*)-caronaldehydic acid and 1.6 g of cyclopropylmethoxyamine hydrochloride in 50 ml of water was stirred at room temperature for about 4 hours in the presence of 2.2 g of sodium bicarbonate. The resulting mixture was filtered through celite and the filtrate was acidified to pH 4 with concentrated hydrochloric acid. The resulting solution was extracted with methylene chloride and the combined extracts were dried over magnesium sulfate and stripped to give 2.0 g of the desired product as an oil; $[\alpha]_D^{25}$ + 30.0° ($CHCl_3$; c = 0.02 g/cc).

Example 22

Alpha-cyano-3-phenoxybenzyl (1R,*cis*)-2,2-dimethyl-3-((cyclopropylmethoxyimino)methyl)-cyclopropanecarboxylate

A solution of 1.9 g of (1R,*cis*)-2,2-dimethyl-3-((cyclopropylmethoxyimino)methyl)cyclopropanecarboxylic acid, 2.6 g of alpha-cyano-3-phenoxybenzyl bromide, 0.9 g of triethylamine and 25 ml of ethyl acetate was heated on a steam bath for three hours and allowed to stand at room temperature for two days. The reaction mixture was diluted with diethyl ether and washed with water. The ether phase was dried over magnesium sulfate and stripped to give 3.8 g of an amber oil. This oil was chromatographed on a three foot silica gel column with a mixture of pentane-ether (9:1 ratio) as eluant to give 2.6 g of desired product as a yellow oil; $[\alpha]_D^{25}$ + 11.2° ($CHCl_3$; c = 0.02 g/cc).

Example 23

3-Phenoxybenzyl (1R,*cis*)-2,2-dimethyl-3-((cyclopropylmethoxyimino)methyl)-cyclopropanecarboxylate

Following a procedure similar to that of Examples 4 and 22 above, 2.3 g of 3-phenoxybenzyl bromide was treated with 1.8 g of (1R,*cis*)-2,2-dimethyl-3-((cyclopropylmethoxyimino)methyl)cyclopropanecarboxylic acid to yield (a) 1.9 g of the desired carboxylate product, as a colourless oil, as a mixture of E- and Z-isomers; $[\alpha]_D^{25}$ + 12.5° ($CHCl_3$; c = 0.02 g/cc).

1.5 g of this product was rechromatographed by the same procedure to yield two products determined by NMR as (b) 0.5 g of the E-isomer as the product having the higher $R_f$ value, and (c) 0.6 g of the Z-isomer as the product having the lower $R_f$ value.

Example 24

Alpha-cyano-3-phenoxybenzyl (1R,*cis*)-2,2-dimethyl-3-((2,2-dichlorocyclopropylmethoxyimino)-methyl)cyclopropanecarboxylate

Following a procedure similar to that of Example 22 above, 1.4 g of alpha-cyano-3-phenoxybenzyl bromide was treated with (1R,*cis*)-2,2-dimethyl-3-((2,2-dichlorocyclopropylmethoxyimino)methyl)-cyclopropanecarboxylic acid (prepared from (1R,*cis*)-caronaldehydic acid and 2,2-dichlorocyclopropylmethoxyamine hydrochloride as in Example 21) to yield 2.2 g of desired carboxylate product as a pale yellow oil; $[\alpha]_D^{25}$ + 12.5° ($CHCl_3$; c = 0.2 g/cc).

Example 25

Diastereoisomers of alpha-cyano-3-phenoxybenzyl (1R,*cis*)-2,2-dimethyl-3-((isopropoxyimino)-methyl)cyclopropanecarboxylate

An isomer mixture of R,S-alpha-cyano-3-phenoxybenzyl (1R,*cis*)-2,2-dimethyl-3-((isopropoxyimino)methyl)cyclopropanecarboxylate (E—Z mixture), prepared as in Example 6 above, was separated into its four (4) diastereoisomer forms using a preparative scale liquid chromatograph (LC). The chromatographic system employed was:

Column — porisil polar bonded phase 9.2 x 250 mm
Mobile Phase — 8% v/v diethyl ether in n-hexane
Flow Rate — 2.5 ml/min
Detection — $UV_{254}$ at AUFS
Injection — typically 500 ml of a 20 mg/ml solution in the mobile phase

The individual diasteroisomers where isolated in greater than 90% purity (as determined by NMR analysis) and are set forth in Table 2 below in terms of their stereoisomeric configuration and column retention time.

Table 2

Diastereoisomers of alpha-cyano-3-phenoxybenzyl (1R,*cis*)-2,2-dimethyl-3-((isopropoxyimino)-methyl)cyclopropanecarboxylate

| Example | Configuration: Oxime | Configuration: Alcohol | Retention Time (min) |
|---|---|---|---|
| 25a | E | R | 6.6 |
| 25b | E | S | 7.8 |
| 25c | Z | R | 7.2 |
| 25d | Z | S | 8.4 |

Following procedures similar to Example 25 above: alpha-cyano-3-phenoxybenzyl (1R,*cis*)-2,2-dimethyl-3-((n-butoxyimino)methyl)cyclopropanecarboxylate, alpha-cyano-3-phenoxybenzyl (1R,*cis*)-2,2 - dimethyl - 3 - ((n - pentoxyimino)methyl)cyclopropanecarboxylate, alpha-cyano-3-phenoxybenzyl (1R,*cis*)-2,2-dimethyl-3-((cyclopentoxyimino)methylcyclopropanecarboxylate, alpha-cyano-3-phenoxybenzyl (1R,*cis*)-2,2-dimethyl-3-((n-propoxyimino)methyl)cyclopropanecarboxylate, and alpha-cyano-3-phenoxybenzyl (1R,*cis*) - 2,2 - dimethyl - 3 - ((isopropoxyimino)methyl)cyclopropanecarboxylate are separated into their individual diastereoisomers.

Example 26
Pesticidal Activity

Activity of the compounds of this invention with respect to insect and acarine pests was determined by using standardised test methods to test the toxicity of the compounds as follows:

I. Houseflies (*Musca domestica* (Linne)) were tested by placing 50 4- to 5-day old houseflies into a spray cage and spraying with 0.6 ml of a solution of test compound. After spraying, the flies were anesthetised with $CO_2$ and transferred to a recovery cage containing a milk pad for food. The cages were held for 18—20 hours after which mortality counts were made. Both dead and moribund were counted. The tests were conducted employing several different dosage rates of each test compound.

II. Pea aphids (*Acyrthosiphon pisum* (Harris)) were tested by placing about 100 aphids on broad bean plants. The plants were sprayed with dilutions of acetone solution of test compound into water containing an emulsifier and held in containers under laboratory conditions for 18 to 20 hours at which time the living aphids in the containers were counted. The tests were conducted employing several different dosage rates of each test compound.

III. Adult female two-spotted spider mites (*Tetranychus urticae* (Koch)) were tested by placing 50—75 mites on the bottom side of leaves of pinto bean plants. The leaves were sprayed with dilutions of acetone solution of test compound into water containing an emulsifier and kept under laboratory conditions for about 20 hours at which time mortality counts were made. The tests were conducted employing several different dosage rates of test compounds.

IV. Mosquito larvae (*Anopheles albimanus* (Weide)) were tested by placing ten living and active mosquito larvae in a jar containing a 0.1 ml aliquot of a 1% acetone solution of test compound thoroughly mixed with 100 ml of distilled water. After 18—22 hours, mortality counts were taken. Both dead and moribund larvae were counted as dead. Larvae which did not swim after being prodded with a needle were considered moribund. The tests were conducted employing several different dosage rates for each test compound.

V. Corn earworm larvae (*Heliothis zea* (Boddie)) were tested by spraying a broad bean plant with dilutions of acetone solution of test compound into water containing an emulsifier. Immediately after spraying, 5 larvae were transferred to the plant and held for 44—46 hours, at which time the dead and moribund larvae were counted. The tests were conducted employing several different dosage rates for each test compound.

In each instance, the toxicity of the compound of the invention was compared to that of a standard pesticide (Parathion), its relative toxicity then being expressed in terms of the relationship between the amount of compound of the invention and the amount of standard pesticide required to produce the same percentage (50) of mortality in the test insects or acarine. Assigning the standard pesticide an arbitrary rating of 100, the toxicities of the compounds of the invention were expressed in terms of the toxicity indexes, which compares the toxicity of the compounds of the invention with that of the standard pesticide. That is to say, a test compound having a Toxicity Index of 50 would he half as active, while one having a Toxicity Index of 200 would be twice as active as the standard pesticide.

Results of the above tests are shown in Table 3.

It can be seen that the compounds of the invention exhibit toxicity against the various pests

tested and were particularly effective on corn earworm larvae. Moreover, a number of compounds of the invention are particularly effective for controlling acarines, such as the two-spotted spider mites as indicated by the compound having a Toxicity Index against mites of about 100 or greater, for example, the compounds of Example 18c (the Z-isomer of 3-phenoxybenzyl (1R,*cis*)-2,2-dimethyl-3-((isobutoxyimino)methyl)cyclopropanecarboxylate) and Example 23c (the Z-isomer of 3-phenoxybenzyl (1R,*cis*)-2,2-dimethyl-3-((cyclopropylmethoxyimino)methyl)cyclopropanecarboxylate).

TABLE 3 — Pesticidal Activity of oxyimino-substituted-(1R, *cis*)-cyclopropanecarboxylates expressed as toxicity index relative to that of parathion as a standard equal to 100

| Example | House Fly | Pea Aphid | 2-Spotted Mite | Mosquito Larvae | Corn Earworm |
|---|---|---|---|---|---|
| 5 | 100 | 25 | + | 17 | 150 |
| 6 | 210 | 370 | 26 | — | 550 |
| 7 | 70 | 120 | 31 | — | 230 |
| 8 | 57 | 54 | 10 | — | 120 |
| 9 | — | 120 | 8 | — | 580 |
| 10 | — | 140 | 55 | — | 670 |
| 4 | 81 | 43 | 22 | — | 130 |
| 12 | 140 | 20 | 5 | 26 | 110 |
| 3 | 65 | 100 | 5 | 170 | 330 |
| 25b | — | — | — | — | 1,700 |
| 25d | — | — | — | — | 2,400 |
| 11 | 140 | 84 | 5 | 100 | 100 |
| 13 | 270 | 250 | 20 | 1,400 | 300 |
| 16 | 650 | 450 | 55 | 4,300 | 390 |
| 2 | 760 | 1,300 | 80 | 3,500 | 1,100 |

— indicates no test

+ Toxicity Index means $>0$ but $<1$

TABLE 3 – Pesticidal activity of oxyimino-substituted-(1R, *cis*)-cyclopropanecarboxylates (Continued)

| Example | House Fly | Pea Aphid | 2-Spotted Mite | Mosquito Larvae | Corn Earworm |
|---|---|---|---|---|---|
| 14 | 520 | 690 | 34 | 4,200 | 920 |
| 15 | 110 | 440 | 82 | 500 | 470 |
| 22 | 480 | 2,500 | 40 | 2,200 | 1,800 |
| 19 | 300 | 440 | 69 | 450 | 330 |
| 20 | 440 | 690 | 140 | 3,000 | 550 |
| 18a | – | – | – | – | 560 |
| 18b | 41 | 48 | 40 | 24 | 230 |
| 18c | 290 | 230 | 310 | 360 | 1,300 |
| 23a | 290 | 100 | 47 | 110 | 440 |
| 23b | 50 | 33 | 25 | 35 | 310 |
| 23c | 490 | 200 | 100 | 480 | 790 |
| 24 | 70 | 570 | 74 | 390 | 480 |

– indicates no test

Moreover, the (1R,*cis*)-compounds of the invention have been found to be unexpectedly more active in the control of certain pests, particularly the corn earworm larvae and mites, than the corresponding structurally most similar (1R,*trans*)-compounds specifically disclosed in U.S. patent 2,922,269. Examples of such esters of the 2,2-dimethyl-3-((oxyimino)methyl)cyclopropanecarboxylic acid are compared in Tables 4, 5, and 6 below, expressed in terms of Toxicity Index relative to parathion as a standard equal to 100.

TABLE 4 – Pesticidal activity of alpha-cyano-3-phenoxybenzyl 3,3-dimethyl-3-((oxyimino) methyl)-cyclopropanecarboxylates expressed as toxicity index relative to the of parathion as a standard equal to 100

| R' | Configuration of Acid | E/Z Isomer Contenr | House Fly | Pea Aphid | Corn Earworm | 2-Spotted Mite | Mosquito Larvae |
|---|---|---|---|---|---|---|---|
| $-CH_2$ 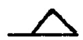 | 1R,*cis* 1) | E–Z mixture | 480 | 2,500 | 1,800 | 40 | 2,200 |
| $-CH_2CH(CH_3)_2$ | 1R, *cis* 2) | E–Z mixture | 760 | 1,300 | 1,100 | 80 | 3,500 |
| $-CH_3$ | 1R, *cis* 3) | E–Z mixture | 98 | 25 | 150 | + | 17 |
| $-CH_3$ | 1R, *trans* 4) | Z isomer | 35 | 24 | 12 | 0 | 23 |
| $-CH_3$ | 1R, *trans* 4) | E isomer | 73 | 28 | 27 | 0 | 96 |

1) Example 22

2) Example 2

3) Example 5

4) Compound named in Example 5 of U.S. 3,922,269

\+ Toxicity Index means >0 but +1

TABLE 5 – Pesticidal activity of 3-phenoxybenzyl 3,3-dimethyl-3-((oxyimino) methyl) cyclopropanecarboxylates expressed as toxicity index relative to that of parathion as a standard equal to 100

| R′ | Configuration of Acid | E/Z Isomer Content | House Fly | Pea Aphid | Corn Earworm | 2-Spotted Mite | Mosquito Larvae |
|---|---|---|---|---|---|---|---|
| $-CH_2$ 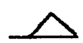 | 1R, *cis* 1) | Z isomer | 490 | 200 | 790 | 100 | 480 |
| $-CH_2CH(CH_3)_2$ | 1R, *cis* 2) | Z isomer | 290 | 230 | 1,300 | 310 | 360 |
| $-CH_3$ | 1R, *cis* 3) | E–Z mixture | 140 | 20 | 110 | 5 | 26 |
| $-CH_3$ | 1R, *trans* 4) | 80% E isomer/ 20% Z isomer | 78 | 3.8 | 23 | 0 | 5.4 |
| $-CH_3$ | 1R, *trans* 4) | 85% Z isomer/ 15% E isomer | 66 | 3.2 | 28 | 0 | 4.2 |

1) Example 23c

2) Example 18c

3) Example 12

4) Compound named in Example 6 of U.S. 3,922,269

TABLE 6 – Pesticidal activity of 5-benzyl-3-furylmethyl 3,3-dimethyl-3-( (oxyimino) cyclopropanecarboxylates expressed as toxicity index relative to that of parathion as a standard equal to 100

| R' | Configuration of Acid | E/Z Isomer Content | House Fly | Pea Aphid | Corn Earworm | 2-Spotted Mite | Mosquito Larvae |
|---|---|---|---|---|---|---|---|
| $-CH_3$ | 1R, *cis* 1) | 55% Z isomer/ 45% E isomer | 65 | 100 | 330 | 5 | 173 |
| $-CH_3$ | 1R, *trans* 2) | 60% Z isomer/ 40% E isomer | 320 | 44 | 140 | 1 | 76 |

1) Example 3

2) Compound named in Example 4 of U.S. 3,922,269

**Claims for the Contracting States: BE CH DE FR GB IT LU NL SE**

1. A method of combating acarine pests at a locus which comprises applying to the acarine pests or to the locus a pesticidally-effective amount of a (1R,*cis*)-oxyimino-substituted-cyclopropane-carboxylate ester, substantially free of other stereoisomers, of the formula:

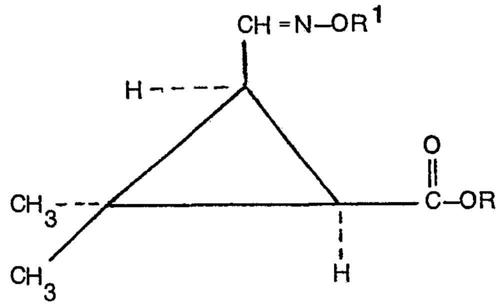

(A)

wherein $R^1$ represents a hydrogen atom, an alkyl group containing 1 to 10 carbon atoms optionally substituted by one or more halogen atoms, a (cycloalkyl)alkyl group containing 3 to 7 ring carbon atoms, a total of 4 to 9 carbon atoms and optionally ring-substituted by one or more halogen atoms, a cycloalkyl group containing 3 to 7 ring carbon atoms, an alkenyl group containing 2 to 4 carbon atoms optionally substituted by one or more halogen atoms or alkynyl group containing 2 to 4 carbon atoms, or an aryl group containing 6 to 12 carbon atoms, or an aralkyl group containing 7 to 10 carbon atoms, each optionally ring-substituted by one or more halogen atoms; $R_7$ is a group of the formula

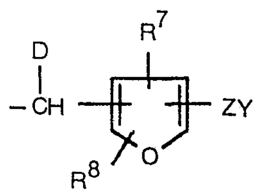

or

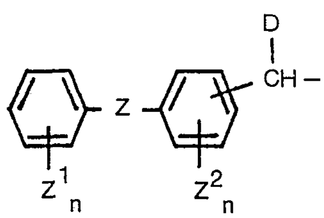

I IV

wherein Y represents a hydrogen atom or a phenyl group which is unsubstituted or substituted in the ring by methyl, methoxy or chlorine, $R^7$ and $R^8$, which may be the same or different, each represent a hydrogen atom or a methyl group, D represents H, —CN, —C≡CH or

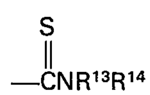

in which $R^{13}$ and $R^{14}$ may be the same or different, each represent a hydrogen atom or an alkyl group containing from 1 to 10 carbon atoms, Z represents $—CH_2—$, —O—, —CO—, or —S—, $Z^1$ and $Z^2$, which may be the same or different, each represent halogen or an alkyl group containing 1 to 4 carbon atoms and n is 0, 1 or 2.

2. A method according to claim 1 characterised in that, in the compound of formula A, $R^1$ is an alkyl group containing 1 to 6 carbon atoms, a (cycloalkyl)alkyl group containing 3 to 6 ring carbon atoms and 4 to 8 total carbon atoms, a cycloalkyl group containing 3 to 6 carbon atoms, an alkenyl or alkynyl group containing 2 to 4 carbon atoms, an aryl group containing from 6 to 12 carbon atoms or an aralkyl group containing 7 to 10 carbon atoms.

3. A method according to claim 2 characterised in that, in the compound of formula A, R is 3-phenoxybenzyl, alpha-cyano-3-phenoxybenzyl or 5-benzyl-3-furylmethyl.

4. A method according to claim 3 characterised in that, in the compound of formula A, $R^1$ is an alkyl group containing 2 to 6 carbon atoms, (cycloalkyl)alkyl containing 3 to 6 ring carbon atoms and 4 to 8 total carbon atoms, allyl, phenyl, benzyl or cycloalkyl containing 3 to 6 carbon atoms.

5. A method according to claim 4 characterised in that, in the compound of formula A, R is alpha-cyano-3-phenoxybenzyl or 3-phenoxybenzyl and $R^1$ is an alkyl, (cycloalkyl)alkyl or cycloalkyl group containing 4 or 5 carbon atoms.

6. A method according to any one of the preceding claims characterised in that the compound of formula A is substantially in the Z-isomer form.

7. A compound of formula A characterised in that $R^1$ is cyclopropyl-methyl or isobutyl, R is 3-phenoxybenzyl and the compound consists substantially of the Z-isomer form of the (1R,*cis*)-oxyimino-substituted-cyclopropanecarboxylate ester, substantially free of other stereoisomers.

**Claims for the Contracting State: AT**

1. A method of combating acarine pests at a locus which comprises applying to the acarine pests or to the locus a pesticidally-effective amount of a (1R,*cis*)-oxyimino-substituted-cyclopropanecarboxylate ester, substantially free of other stereoisomers, of the formula:

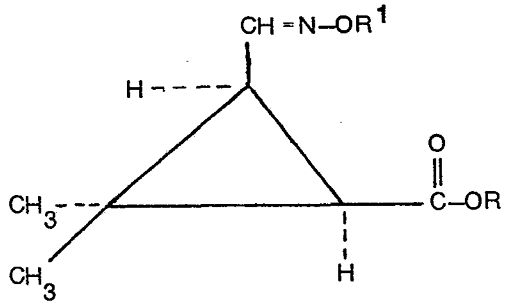

(A)

wherein $R^1$ represents a hydrogen atom, an alkyl group containing 1 to 10 carbon atoms optionally substituted by one or more halogen atoms, a (cycloalkyl)alkyl group containing 3 to 7 ring carbon atoms, a total of 4 to 9 carbon atoms and optionally ring-substituted by one or more halogen atoms, a cycloalkyl group containing 3 to 7 ring carbon atoms, an alkenyl group containing 2 to 4 carbon atoms optionally substituted by one or more halogen atoms or alkynyl group containing 2 to 4 carbon atoms or an aryl group containing 6 to 12 carbon atoms, or an aralkyl group containing 7 to 10 carbon atoms, each optionally ring-substituted by one or more halogen atoms; R is a group of the formula

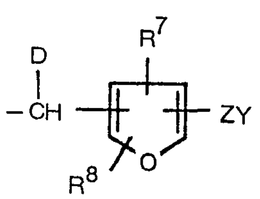

I

or

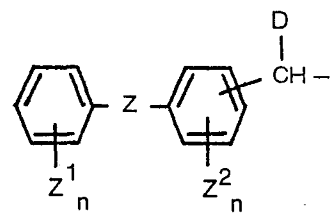

IV

wherein Y represents a hydrogen atom or a phenyl group which is unsubstituted or substituted in the ring by methyl, methoxy or chlorine, $R^7$ and $R^8$, which may be the same or different, each represent a hydrogen atom or a methyl group, D represents H, —CN, —C≡CH or

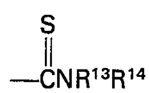

in which $R^{13}$ and $R^{14}$ may be the same or different, each represent a hydrogen atom or an alkyl group containing from 1 to 10 carbon atoms, Z represents $—CH_2—$, —O—, —CO—, or —S—, $Z^1$ and $Z^2$, which may be the same or different, each represent halogen or an alkyl group containing 1 to 4 carbon atoms and n is 0, 1 or 2.

2. A method according to claim 1 characterised in that, in the compound of formula A, $R^1$ is an alkyl group containing 1 to 6 carbon atoms, a (cycloalkyl)alkyl group containing 3 to 6 ring carbon atoms and 4 to 8 total carbon atoms, a cycloalkyl group containing 3 to 6 carbon atoms, an alkenyl or alkynyl group containing 2 to 4 carbon atoms, an aryl group containing from 6 to 12 carbon atoms or an aralkyl group containing 7 to 10 carbon atoms.

3. A method according to claim 2 characterised in that, in the compound of formula A, R is 3-phenoxybenzyl, alpha-cyano-3-phenoxybenzyl or 5-benzyl-3-furylmethyl.

4. A method according to claim 3 characterised in that, in the compound of formula A, $R^1$ is an alkyl group containing 2 to 6 carbon atoms, (cycloalkyl)alkyl containing 3 to 6 ring carbon atoms and 4 to 8 total carbon atoms, allyl, phenyl, benzyl or cycloalkyl containing 3 to 6 carbon atoms.

5. A method according to claim 4 characterised in that, in the compound of formula A, R is alpha-cyano-3-phenoxybenzyl or 3-phenoxybenzyl and $R^1$ is an alkyl, (cycloalkyl)alkyl or cycloalkyl group containing 4 or 5 carbon atoms.

6. A method according to any one of the preceding claims characterised in that the compound of formula A is substantially in the Z-isomer form.

**Patentansprüche für die Vertragsstaaten: BE CH DE FR GB IT LU NL SE**

1. Eine Methode zur Bekämpfung von Akarid-Schädlingen an einem Ort, dadurch gekennzeichnet, daß man auf die Akarid-Schädlinge oder auf den Ort eine pestizid wirksame Menge eines (1R,cis)-Oxyimino-substituierten Cyclopropancarboxylat-esters, der im wesentlichen frei von anderen Stereoisomeren ist, entsprechend der Formel

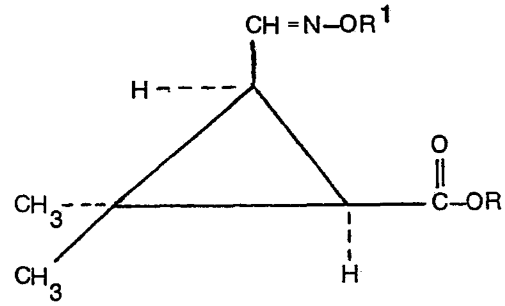

(A)

aufbringt, worin $R^1$ ein Wasserstoffatom, eine Alkylgruppe mit 1 bis 10 Kohlenstoffatomen, die gegebenenfalls durch eine oder mehrere Halogenatome substituiert ist, eine (Cycloalkyl)alkylgruppe mit 3 bis 7 Ringkohlenstoffatomen und insgesamt 4 bis 9 Kohlenstoffatomen, die gegebenenfalls durch ein oder durch mehrere Halogenatome substituiert ist, eine Cycloalkylgruppe mit 3 bis 7 Ringkohlenstoffatomen, eine Alkenylgruppe mit 2 bis 4 Kohlenstoffatomen, die gegebenenfalls durch ein oder durch mehrere Halogenatome substituiert ist, oder eine Alkinylgruppe mit 2 bis 4 Kohlenstoffatomen oder eine Arylgruppe mit 6 bis 12 Kohlenstoffatomen oder eine Aralkylgruppe mit 7 bis 10 Kohlenstoffatomen, die jeweils gegebenenfalls durch ein oder durch mehrere Halogenatome ringsubstituiert sind, bedeutet; R eine Gruppe der Formel

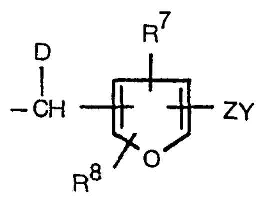

(I)

oder

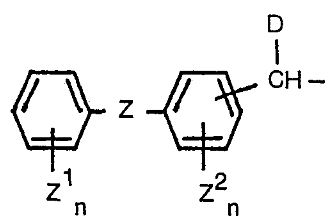

(IV)

darstellt, worin Y ein Wasserstoffatom oder eine Phenylgruppe bedeutet, die unsubstituiert oder im Ring durch Methyl, Methoxy oder Chlor substituiert ist, $R^7$ und $R^8$, die gleich oder verschieden sein können, jeweils für ein Wasserstoffatom oder eine Methylgruppe stehen, D für Wasserstoff, —CN, —C≡CH oder

$$-\overset{S}{\overset{\|}{C}}NR^{13}R^{14}$$

steht, worin $R^{13}$ und $R^{14}$ gleich oder verschieden sein können und jeweils für ein Wasserstoffatom oder eine Alkylgruppe mit 1 bis 10 Kohlenstoffatomen stehen, Z für $—CH_2—$, —O—, —CO— oder —S— steht, $Z^1$ und $Z^2$, die gleich oder verschieden sein können, jeweils Halogen oder eine Alkylgruppe mit 1 bis 4 Kohlenstoffatomen bedeuten und n für Null, 1 oder 2 steht.

2. Eine Methode nach Anspruch 1, dadurch gekennzeichnet, daß in der Verbindung der Formel (A) $R^1$ eine Alkylgruppe mit 1 bis 6 Kohlenstoffatomen, eine (Cycloalkyl)alkylgruppe mit 3 bis 6 Ringkohlenstoffatomen und insgesamt 4 bis 8 Kohlenstoffatomen, eine Cycloalkylgruppe mit 3 bis 6 Kohlenstoffatomen, eine Alkenyl- oder Alkinylgruppe mit 2 bis 4 Kohlenstoffatomen, eine Arylgruppe mit 6 bis 12 Kohlenstoffatomen oder eine Aralkylgruppe mit 7 bis 10 Kohlenstoffatomen bedeutet.

3. Eine Methode nach Anspruch 2, dadurch gekennzeichnet, daß in der Verbindung der Formel (A) R für 3-Phenoxybenzyl, α-Cyano-3-phenoxybenzyl oder 5-Benzyl-3-furylmethyl steht.

4. Eine Methode nach Anspruch 3, dadurch gekennzeichnet daß in der Verbindung der Formel (A) $R^1$ für eine Alkylgruppe mit 2 bis 6 Kohlenstoffatomen, (Cycloalkyl)alkyl mit 3 bis 6 Ringkohlenstoffatomen und insgesamt 4 bis 8 Kohlenstoffatomen, Allyl, Phenyl, Benzyl oder Cycloalkyl mit 3 bis 6 Kohlenstoffatomen steht.

5. Eine Methode nach Anspruch 4, dadurch gekennzeichnet, daß in der Verbindung der Formel (A) R für $\alpha$-Cyano-3-phenoxybenzyl oder 3-Phenoxybenzyl steht und $R^1$ eine Alkyl-(Cycloalkyl)alkyl- oder Cycloalkylgruppe mit 4 oder 5 Kohlenstoffatomen steht.

6. Ein Verfahren nach einem der vorstehenden Ansprüche, dadurch gekennzeichnet, daß die Verbindung der Formel (A) im wesentlichen in Form des Z-Isomers vorliegt.

7. Eine Verbindung der Formel (A), dadurch gekennzeichnet, daß $R^1$ für Cyclopropylmethyl oder Isobutyl steht, R 3-Phenoxybenzyl bedeutet und die Verbindung im wesentlichen aus der Z-Isomerform des (1R,cis-)-Oxyimino-substituierten Cyclopropancarboxylatesters besteht und im wesentlichen frei von anderen Stereoisomeren ist.

**Patentansprüche für den Vertragsstaat: AT**

1. Eine Methode zur Bekämpfung von Akarid-Schädlingen and einem Ort, dadurch gekennzeichnet, daß man auf die Akarid-Schädlinge oder auf den Ort eine pestizid wirksame Menge eines (1R,cis)-Oxyimino-substituierten Cyclopropancarboxylatesters, der im wesentlichen frei von anderen Stereoisomeren ist, entsprechend der Formel

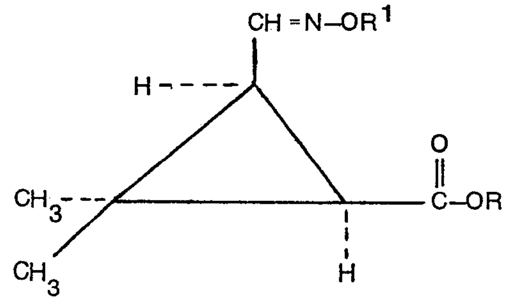

(A)

aufbringt, worin $R^1$ ein Wasserstoffatom, eine Alkylgruppe mit 1 bis 10 Kohlenstoffatomen, die gegebenenfalls durch eine oder mehrere Halogenatome substituiert ist, eine (Cycloalkyl)alkylgruppe mit 3 bis 7 Ringkohlenstoffatomen und insgesamt 4 bis 9 Kohlenstoffatomen, die gegebenenfalls durch ein oder durch mehrere Halogenatome substituiert ist, eine Cycloalkylgruppe mit 3 bis 7 Ringkohlenstoffatomen, eine Alkenylgruppe mit 2 bis 4 Kohlenstoffatomen, die gegebenenfalls durch ein oder durch mehrere Halogenatome substituiert ist, oder eine Alkinylgruppe mit 2 bis 4 Kohlenstoffatomen oder eine Arylgruppe mit 6 bis 12 Kohlenstoffatomen oder eine Aralkylgruppe mit 7 bis 10 Kohlenstoffatomen, die jeweils gegebenenfalls durch ein oder durch mehrere Halogenatome ringsubstituiert sind, bedeutet; R eine Gruppe der Formel

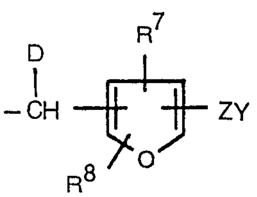

(I)

oder

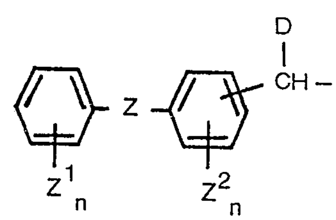

(IV)

darstellt, worin Y ein Wasserstoffatom oder eine Phenylgruppe bedeutet, die unsubstituiert oder im Ring durch Methyl, Methoxy oder Chlor substituiert ist, $R^7$ und $R^8$, die gleich oder verschieden sein können, jeweils für ein Wasserstoffatom oder eine Methylgruppe stehen, D für Wasserstoff, —CN, —C≡CH oder

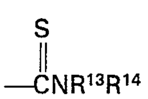

steht, worin $R^{13}$ und $R^{14}$ gleich oder verschieden sein können und jeweils für ein Wasserstoffatom oder eine Alkylgruppe mit 1 bis 10 Kohlenstoffatomen stehen, Z für $—CH_2—$, —O—, —CO— oder —S— steht, $Z^1$ und $Z^2$, die gleich oder verschieden sein können, jeweils Halogen oder eine Alkylgruppe mit 1 bis 4 Kohlenstoffatomen bedeuten und n für Null, 1 oder 2 steht.

2. Eine Methode nach Anspruch 1, dadurch gekennzeichnet, daß in der Verbindung der Formel (A)

$R^1$ eine Alkylgruppe mit 1 bis 6 Kohlenstoffatomen, eine (Cycloalkyl)alkylgruppe mit 3 bis 6 Ringkohlenstoffatomen und insgesamt 4 bis 8 Kohlenstoffatomen, eine Cycloalkylgruppe mit 3 bis 6 Kohlenstoffatomen, eine Alkenyl- oder Alkinylgruppe mit 2 bis 4 Kohlenstoffatomen, eine Arylgruppe mit 6 bis 12 Kohlenstoffatomen oder eine Aralkylgruppe mit 7 bis 10 Kohlenstoffatomen bedeutet.

3. Eine Methode nach Anspruch 2, dadurch gekennzeichnet, daß in der Verbindung der Formel (A) R für 3-Phenoxybenzyl, $\alpha$-Cyano-3-phenoxybenzyl oder 5-Benzyl-3-furylmethyl steht.

4. Eine Methode nach Anspruch 3, dadurch gekennzeichnet, daß in der Verbindung der Formel (A) $R^1$ für eine Alkylgruppe mit 2 bis 6 Kohlenstoffatomen, (Cycloalkyl)alkyl mit 3 bis 6 Ringkohlenstoffatomen und insgesamt 4 bis 8 Kohlenstoffatomen, Allyl, Phenyl, Benzyl oder Cycloalkyl mit 3 bis 6 Kohlenstoffatomen steht.

5. Eine Methode nach Anspruch 4, dadurch gekennzeichnet, daß in der Verbindung der Formel (A) R für $\alpha$-Cyano-3-phenoxybenzyl oder 3-Phenoxybenzyl steht und $R^1$ eine Alkyl-, (Cycloalkyl)alkyl- oder Cycloalkylgruppe mit 4 oder 5 Kohlenstoffatomen steht.

6. Ein Verfahren nach einem der vorstehenden Ansprüche, dadurch gekennzeichnet, daß die Verbindung der Formel (A) im wesentlichen in Form des Z-Isomers vorliegt.

**Revendications pour les Etats contractants: BE CH DE FR GB IT LU NL SE**

1. Une méthode de lutte contre les acariens nuisibles en un lieu qui comprend l'application aux acariens nuisibles ou au lieu à traiter d'une quantité efficace du point de vue pesticide d'un ester d'acide (1R,*cis*)-cyclopropanecarboxylique à substituant oxyimino, sensiblement exempt d'autres stéréoisomères, de la formule:

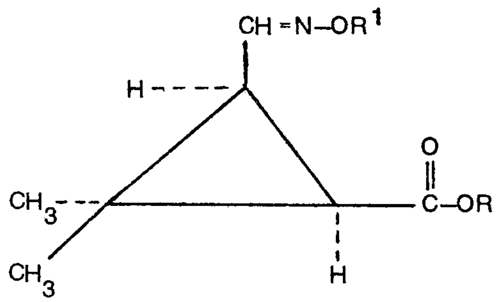

(A)

dans laquelle $R^1$ représente un atome d'hydrogène, un groupe alcoyle contenant de 1 à 10 atomes de carbone éventuellement substitué par un ou plusieurs atomes d'halogènes, un groupe (cycloalcoyl)alcoyle contenant de 3 à 7 atomes de carbone dans le noyau, un total de 4 à 9 atomes de carbone et éventuellement substitué au noyau par un ou plusieurs atomes d'halogènes, un groupe cycloalcoyle contenant de 3 à 7 atomes de carbone dans le noyau, un groupe alcényle contenant de 2 à 4 atomes de carbone éventuellement substitué par un ou plusieurs atomes d'halogènes ou un groupe alcynyle contenant 2 à 4 atomes de carbone ou un groupe aryle contenant 6 à 12 atomes de carbone ou un groupe aralcoyle contenant 7 à 10 atomes de carbone, chacun éventuellement substitué au noyau par un ou plusieurs atomes d'halogènes; R est un groupe de la formule

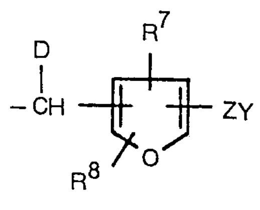

I

ou

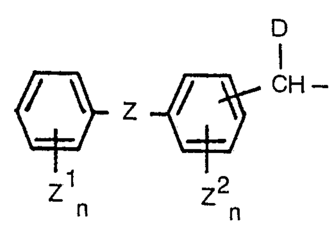

IV

où Y représente un atome d'hydrogène ou un groupe phényle qui est non-substitué ou substitué au noyau par un groupe méthyle ou méthoxy ou par du chlore, $R^7$ et $R^8$, qui peuvent être identiques ou différents, représentent chacun un atome d'hydrogène ou un groupe méthyle, D représente H, —CN, —C≡CH ou

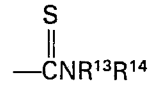

où $R^{13}$ et $R^{14}$ peuvent être identiques ou différents et représentent chacun un atome d'hydrogène ou un groupe alcoyle contenant de 1 à 10 atomes de carbone, Z représente $—CH_2—$, —O—, —CO— ou —S—, $Z^1$ et $Z^2$, qui peuvent être identiques ou différents, représentent chacun un halogène ou un groupe alcoyle contenant de 1 à 4 atomes de carbone et n est 0, 1 ou 2.

2. Une méthode selon la revendication 1, caractérisée en ce que, dans le composé de formule A, $R^1$ est un groupe alcoyle contenant 1 à 6 atomes de carbone, un groupe (cycloalcoyl)alcoyle contenant 3 à 6 atomes de carbone dans le noyau et 4 à 8 atomes de carbone au total, un groupe cycloalcoyle contenant 3 à 6 atomes de carbone, un groupe alcényle ou alcynyle contenant 2 à 4 atomes de carbone, un groupe aryle contenant de 6 à 12 atomes de carbone ou un groupe aralcoyle contenant 7 à 10 atomes de carbone.

3. Une méthode selon la revendication 2 caractérisée en ce que, dans le composé de formule A, R est un groupe 3-phénoxybenzyle, alpha-cyano-3-phénoxybenzyle ou 5-benzyl-3-furylméthyle.

4. Une méthode selon la revendication 3, caractérisée en ce que, dans le composé de formule A, $R^1$ est un groupe alcoyle contenant 2 à 6 atomes de carbone, (cycloalcoyl)alcoyle contenant 3 à 6 atomes de carbone dans le noyau et 4 à 8 atomes de carbone au total, allyle, phényle, benzyle ou cycloalcoyle contenant 3 à 6 atomes de carbone.

5. Une méthode selon la revendication 4, caractérisée en ce que, dans le composé de formule A, R est un groupe alpha-cyano-3-phénoxybenzyle ou 3-phénoxybenzyle et $R^1$ est un groupe alcoyle, (cycloalcoyl)alcoyle ou cycloalcoyle contenant 4 ou 5 atomes de carbone.

6. Une méthode selon l'une quelconque des revendications précédentes, caractérisée en ce que le composé de formule A est essentiellement sous la forme de l'isomère Z.

7. Un composé de formule A caractérisé en ce que $R^1$ est un groupe cyclopropyl-méthyle ou isobutyle, R est un groupe 3-phénoxybenzyle et le composé est constitué essentiellement de la forme isomère Z du (1R,cis)-cyclopropanecarboxylate à substituant oxyimino, sensiblement exempte d'autres stéréoisomères.

**Revendications pour l'Etat contractant: AT**

1. Une méthode de lutte contre les acariens nuisibles en un lieu qui comprend l'application aux acariens nuisibles ou au lieu à traiter d'une quantité efficace du point de vue pesticide d'un ester d'acide (1R,*cis*)-cyclopropanecarboxylique à substituant oxyimino, sensiblement exempt d'autres stéréoisomères, de la formule:

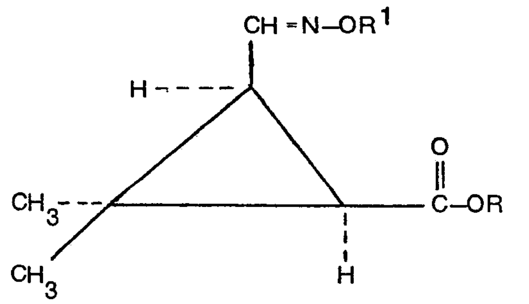

(A)

dans laquelle $R^1$ représente un atome d'hydrogène, un groupe alcoyle contenant de 1 à 10 atomes de carbone éventuellement substitué par un ou plusieurs atomes d'halogènes, un groupe (cycloalcoyl)alcoyle contenant de 3 à 7 atomes de carbone dans le noyau, un total de 4 à 9 atomes de carbone et éventuellement substitué au noyau par un ou plusieurs atomes d'halogènes, un groupe cycloalcoyle contenant de 3 à 7 atomes de carbone dans le noyau, un groupe alcényle contenant de 2 à 4 atomes de carbone éventuellement substitué par un ou plusieurs atomes d'halogènes ou un groupe alcynyl contenant 2 à 4 atomes de carbone ou un groupe aryle contenant 6 à 12 atomes de carbone ou un groupe aralcoyle contenant 7 à 10 atomes de carbone, chacun éventuellement substitué au noyau par un ou plusieurs atomes d'halogènes; R est un groupe de la formule

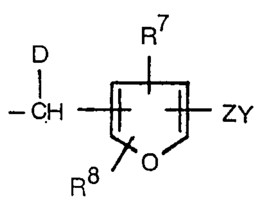

ou

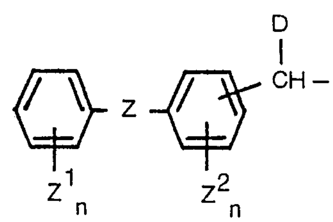

I

IV

où Y représente un atome d'hydrogène ou un groupe phényle qui est non-substitué ou substitué au noyau par un groupe méthyle ou méthoxy ou par du chlore, $R^7$ et $R^8$, qui peuvent être identiques ou différents, représentent chacun un atome d'hydrogène ou un groupe méthyle, D représente H, —CH, —C≡CH ou

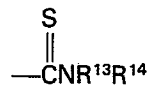

où $R^{13}$ et $R^{14}$ peuvent être identiques ou différents et représentent chacun un atome d'hydrogène ou un groupe alcoyle contenant de 1 à 10 atomes de carbone, Z représente $—CH_2—$, —O—, —CO— ou —S—, $Z^1$ et $Z^2$, qui peuvent être identiques ou différents, représentent chacun un halogène ou un groupe alcoyle contenant de 1 à 4 atomes de carbone et n est 0, 1 ou 2.

2. Une méthode selon la revendication 1, caractérisée en ce que, dans le composé de formule A, $R^1$ est un groupe alcoyle contenant 1 à 6 atomes de carbone, un groupe (cycloalcoyl)alcoyle contenant 3 à 6 atomes de carbone dans le noyau et 4 à 8 atomes de carbone au total, un groupe cycloalcoyle contenant 3 à 6 atomes de carbone, un groupe alcényle ou alcynyle contenant 2 à 4 atomes de carbone, un groupe aryle contenant de 6 à 12 atomes de carbone ou un groupe aralcoyle contenant 7 à 10 atomes de carbone.

3. Une méthode selon la revendication 2 caractérisée en ce que, dans le composé de formule A, R est un groupe 3-phénoxybenzyle, alpha-cyano-3-phénoxybenzyle ou 5-benzyl-3-furylméthyle.

4. Une méthode selon la revendication 3, caractérisée en ce que, dans le composé de formule A, $R^1$ est un groupe alcoyle contenant 2 à 6 atomes de carbone, (cycloalcoyl)alcoyle contenant 3 à 6 atomes de carbone dans le noyau et 4 à 8 atomes de carbone au total, allyle, phényle, benzyle ou cycloalcoyle contenant 3 à 6 atomes de carbone.

5. Une méthode selon la revendication 4, caractérisée en ce que, dans le composé de formule A, R est un groupe alpha-cyano-3-phénoxybenzyle ou 3-phénoxybenzyle et $R^1$ est un groupe alcoyle, (cycloalcoyl)alcoyle ou cycloalcoyle contenant 4 ou 5 atomes de carbone.

6. Une méthode selon l'une quelconque des revendications précédentes, caractérisée en ce que le composé de formule A est essentiellement sous la forme de l'isomère Z.